# EUROPÄISCHE PATENTSCHRIFT

(11) **EP 0 540 974 B1**
(45) Veröffentlichungstag und Bekanntmachung des Hinweises auf die Patenterteilung: **12.07.2000**
(21) Anmeldenummer: 92118288.7
(22) Anmeldetag: 26.10.1992
(51) Int. Cl.: G01N 27/403, G01N 33/00, G01N 27/416, G01N 27/49

(54) **Vorrichtung zum simultanen Nachweis verschiedener Gaskomponenten**
Apparatus for simultaneous determination of different gas components
Appareil pour la détermination simultanée de composants différents d'un gaz

(30) Priorität: 08.11.1991 DE 4136779
(43) Veröffentlichungstag der Anmeldung: 12.05.1993
(73) Patentinhaber: Compur Monitors Sensor Technology GmbH, 81539 München (DE)
(72) Erfinder: Braden, Christoph, Dr., W-5000 Köln 41 (DE); Deprez, Jacques, W-5020 Frechen (DE)
(74) Vertreter: Wagner, Karl H., Dipl.-Ing.

(56) Entgegenhaltungen:
- EP-A- 0 064 337
- EP-A- 0 293 255
- EP-A- 0 293 541
- FR-A- 2 280 079
- GB-A- 2 155 185
- US-A- 3 969 209
- US-A- 4 315 753
- US-A- 4 506 226
- PATENT ABSTRACTS OF JAPAN vol. 012 no. 298 (P-744) ,15.August 1988 & JP-A-63 071649 (FUJITSU LTD) 1.April 1988,

## Beschreibung

Die Erfindung geht aus von einer Vorrichtung zum simultanen Nachweis verschiedener Gaskomponenten mit einer Vielzahl von elektrochemischen Dreielektrodenmeßzellen (Arbeitselektrode-Gegenelektrode-Bezugselektrode) mit einem gemeinsamen Elektrolyt und einer potentiostatischen Auswerteschaltung zur Vorgabe und Regelung unterschiedlicher Potentiale an den Arbeitselektroden und zur Messung der elektrochemisch erzeugten, mit den einzelnen Gaskonzentrationen korrelierten elektrischen Signale.

Potentiostatische Dreielektrodensensoren sind weit verbreitet zur Messung von Gasen im Spurenbereich bis hin zu Untersuchungen an reinen Gasen. Diese Gassensoren sind in der Regel reproduzierbar, empfindlich und für eine Vielzahl von verschiedenen Gasen realisierbar. Die Selektivität kann durch die Wahl des Katalysators an der Meßelektrode, des Elektrolyts und des Potentials an der Meßelektrode (Arbeitselektrode) beeinflußt werden. Jedoch lassen sich nicht alle Querempfindlichkeiten gleichzeitig ausschalten. Vielmehr muß in der Praxis von Anwendungsfall zu Anwendungsfall ein Kompromiß zwischen der Empfindlichkeit und der Unterdrückung von Querempfindlichkeit gegenüber störenden anderen Gasen gefunden werden.

Als Alternative bietet sich an, mehrere Sensoren zu einem Sensor-Array zusammenzufassen und die unterschiedlichen Empfindlichkeiten für die gesuchte Meßkomponente und die störenden Querkomponenten bei der Meßwertverarbeitung zu benutzen. Durch Berücksichtigung aller Sensorsignale läßt sich dann die Zusammensetzung des Gases bestimmen (Mustererkennung). Derartige Sensor-Arrays wurden schon auf der Basis von LeitfähigkeitsFestkörper-Gassensoren realisiert.

Ein elektrochemischer Multielektroden-Sensor, bei dem das Gas durch hintereinander angeordnete elektrochemische Meßzellen strömt, ist in DE 24 35 813 beschrieben. Die Meßzellen sind über ein mit den Elektroden verbundenes Widerstandsnetzwerk derart miteinander verknüpft, daß in jeder Zelle nur eine einem bestimmten Schadstoff zugeordnete Meßspannung gebildet wird. Voraussetzung ist dafür, daß in jeder Stufe eine vollständige Reaktion erfolgt, so daß längere Verweilzeiten und damit auch längere Totzeiten bei der Messung in Kauf genommen werden müssen. Das zugrundeliegende Meßprinzip bedingt ferner, daß sämtliche Elektroden für jede Meßzelle separat herausgeführt sind. Diese Eigenschaften stehen der Forderung nach einem einfach aufgebauten, kompakten elektrochemischen Multielektroden-Sensor entgegen.

Außerdem müssen Temperaturunterschiede und unterschiedliche Anströmungen bei den einzelnen Meßzellen berücksichtigt werden.

Ferner sind aus US 43 15 753 und EP 00 64 337 potentiostatische Vierelektroden-Sensoren (zwei Arbeitselektroden, eine Referenz- und eine Gegenelektrode) zur Messung spezieller Gassysteme bekannt. Es besteht jedoch keine Möglichkeit, die Potentiale der Arbeitselektroden unabhängig voneinander zu wählen. Damit entfällt ein wesentlicher Freiheitsgrad zur individuellen Optimierung der Selektivitäten. Davon abgesehen, ist bei diesem Stand der Technik zum Teil auch eine aufwendige Gasführung erforderlich.

Der Erfindung liegt die Aufgabe zugrunde, mehrere Gaskomponenten gleichzeitig und unabhängig voneinander mit Hilfe eines elektrochemischen Multielektroden-Sensors zu messen, wobei im Hinblick auf eine Optimierung der Selektivität für den Nachweis der einzelnen Gaskomponenten vollkommene Freiheit bei der Festlegung des Elektrodenpotentials, der Auswahl des Elektrodenmaterials einschließlich katalytischer Zusätze und der Verwendung geeigneter Gasfilter und Gasdiffusionsbarrieren herrscht.

Diese Aufgabe wird bei einem elektrochemischen Sensor mit einer Vielzahl von Dreielektrodenmeßzellen erfindungsgemäß dadurch gelöst,
a) daß die Dreielektrodenmeßzellen durch eine Vielzahl von Arbeitselektroden mit einer gemeinsamen Gegenelektrode und einer gemeinsamen Bezugselektrode gebildet werden, die mit demselben Elektrolyt in Verbindung stehen,
b) daß eine potentiostatische Auswerteschaltung Regelkreise enthält, die die Potentiale der Arbeitselektroden, bezogen auf die Bezugselektrode, einzeln und unabhängig voneinander konstant halten
c) und daß die Auswerteschaltung Mittel zur Erfassung der in die Arbeitselektroden fließenden, mit den Gaskonzentrationen korrelierten elektrischen Stromsignale aufweist.

Vorzugsweise besteht ein Regelkreis in der potentiostatischen Auswerteschaltung jeweils aus zwei in Kaskade geschalteten Operationsverstärkern, wobei der erste Verstärker die Potentialdifferenz zwischen einer Arbeitselektrode und der gemeinsamen Bezugselektrode hochohmig abgreift und der zweite Verstärker diese Potentialdifferenz am Ausgang des ersten Verstärkers mit einem voreingestellten Sollwert Uₙ vergleicht und den vom Ausgang des zweiten Verstärkers zur Arbeitselektrode fließenden Strom Iₙ so nachregelt, daß die Abweichung vom Sollwert Uₙ minimiert wird. Die Ströme Iₙ, die sich bei dieser Regelung an den verschiedenen Arbeitselektroden einstellen, sind die Meßwerte für die Gaskonzentrationen der an den Arbeitselektroden eintreffenden Gaskomponenten.

Vorzugsweise wird ein flüssiger Elektrolyt verwendet. Die Arbeitselektroden werden vorteilhaft in Form von räumlich getrennten Meßfeldern auf der einen Oberfläche des Elektrolyten untergebracht, während die gemeinsame Gegenelektrode und die gemeinsame Bezugselektrode auf der gegenüberliegenden Seite des Elektrolyten angeordnet sind. Auf diese Weise läßt sich besonders gut ein kompakter elektrochemischer Multielektroden-Sensor realisieren.

Die Empfindlichkeit einer Arbeitselektrode für ein bestimmtes Gas und damit die Empfindlichkeit eines Meßfeldes für eine bestimmte Gaskomponente, ist in bekannter Weise vom Elektrodenmaterial und vom Elektrodenpotential abhängig und kann damit über das Elektrodenpotential eingestellt werden, Weiterhin kann die Selektivität eines Meßfeldes durch katalytische Aktivierung der Arbeitselektrode und durch Vorschaltung von gasspezifischen Filtern verbessert werden. Ferner kann die Empfindlichkeit einer Meßzelle durch Blenden oder Diffusionsmembranen unterschiedlich eingestellt werden. Ein Multielektroden-Sensor mit unterschiedlichen Empfindlichkeiten der einzelnen Meßfelder erlaubt grundsätzlich eine Mustererkennung und damit die Identifizierung von bestimmten Gasgemischen.

Mit der Erfindung werden folgende Vorteile erzielt:
- Gegenüber den bisher bekannten elektrochemischen Mehrelektroden-Sensoren kann aufgrund der raumsparenden Bauweise eine deutliche Volumenreduzierung erreicht werden, da Bezugselektrode, Gegenelektrode, Elektrolyt und das gesamte Sensorgehäuse für alle Arbeitselektroden gemeinsam genutzt werden.
- Bei Verwendung eines Festkörperelektrolyts ergibt sich eine weitere Volumenreduzierung, wenn der Sensor in Hybridtechnik gefertigt wird.
- Alle Arbeitselektroden werden bei identischen Temperatur-, Druck- und Anströmungsbedingungen betrieben. Auf diese Weise können Störeinflüsse, die auf unterschiedliche Schwankungen dieser Parameter zurückzuführen sind, vermieden werden.
- Alle Meßsignale an den Arbeitselektroden (Arbeitselektrodenströme Iₙ) beziehen sich auf dieselbe Bezugselektrode und denselben Elektrolyten, so daß Potentialdrifts der Bezugselektrode erkannt und gegebenenfalls kompensiert werden können.
- Mit dem erfindungsgemäßen Multielektroden-Sensor kann man ein zyklisch voltametrisches Diagramm (Voltamogramm) mit sehr hoher Auflösung (entsprechend der Anzahl der Arbeitselektroden) momentan und simultan statisch aufnehmen, wohingegen man bei der klassischen zyklischen Voltametrie selbst bei sehr langsamen Messungen (Geschwindigkeiten bis zu 1 mV/min) nur dynamische Grenzwerte erhält.
- Der erfindungsgemäße Multielektroden-Sensor läßt ferner eine gezielte Unterdrückung von Querempfindlichkeiten gegenüber anderen unerwünschten Gaskomponenten zu.
- Da die einzelnen Arbeitselektroden vollkommen unabhängig voneinander potentiostatisch betrieben werden, besteht vollkommene Freiheit bei der Wahl und Einstellung des Elektrodenpotentials, der Auswahl der Katalysatoren an den Arbeitselektroden, der Vorschaltung von gasspezifischen Filtern, so daß die einzelnen Meßzellen mit optimaler Selektivität an das Meßproblem angepaßt werden können.
- Das zu untersuchende Gas steht gleichzeitig an allen Arbeitselektroden an, da die Gaswege gleich sind. Dadurch können unterschiedliche Ansprechzeiten, die auf verschiedene Strömungswege oder Diffusionsstrecken bei den einzelnen Gaskomponenten zurückzuführen sind, vermieden werden.
- Grundsätzlich bestehen bei dem erfindungsgemäßen Multielektroden-Sensor hinsichtlich des Nachweises bestimmter Gaskomponenten oder eines bestimmten Gemisches keine Einschränkungen. So können z.B. feste, flüssige, anorganische oder organische Elektrolyten verwendet werden.

Im folgenden wird ein Ausführungsbeispiel der Erfindung anhand von Zeichnungen näher erläutert. Es zeigen
- Fig. 1: den prinzipiellen Aufbau eines Multielektroden-Sensors mit einer Vielfach-Potentiostatenschaltung zur Regelung der Arbeitselektroden, Potentiale und Messung der in die Arbeitselektroden fließenden Ströme,
- Fig. 2: die praktische Ausführung eines Multielektroden-Sensors,
- Fig. 3a-3c: Ausführungen des Multielektroden-Sensors mit zwei, drei und vier Maßfeldern auf der Gasseite eines flüssigen Elektrolyten,

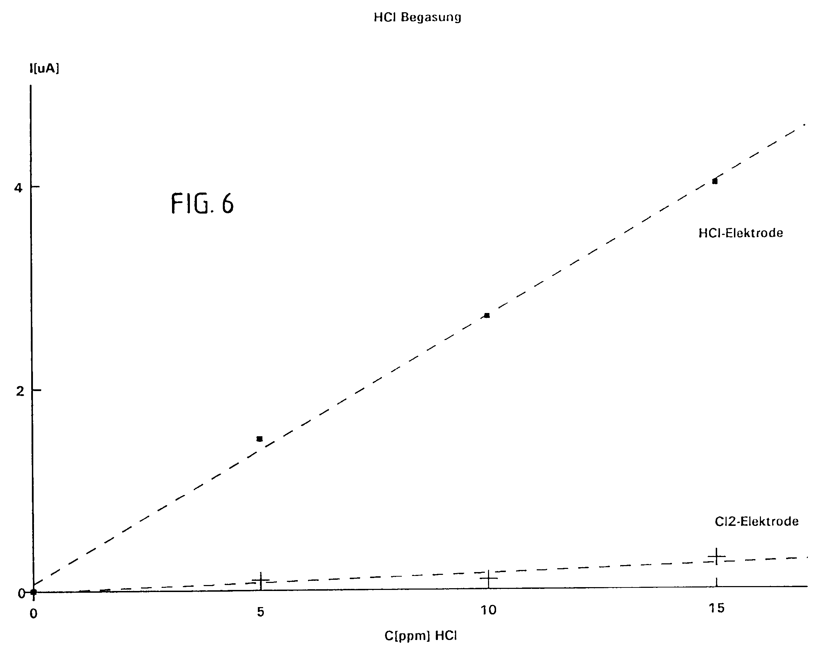

- Fig. 4-6: verschiedene Meßbeispiele zur Erläuterung des Einflusses des Arbeitselektrodenpotentials auf die Selektivität,
- Fig. 7: den zeitlichen Verlauf des Meßsignales (Response-Kurve) an zwei Meßfeldern bei einem Mehrelektroden-Sensor gamäß Fig. 1 bei Begasung mit Cl₂ und HCl und
- Fig. 8: die Response-Kurven bei demselben Mehrelektroden-Sensor bei der Begasung mit Cl₂ und HCl .

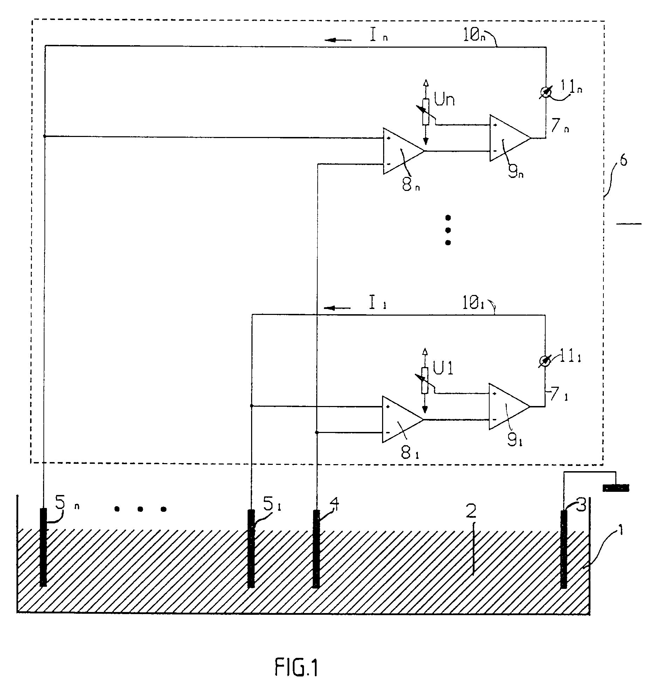

In Fig. 1 ist der Multielektroden-Sensor mit dem Gehäuse 1, dem Meßzellenelektrolyt 2 und den Elektroden nur schematisch dargestellt. Die Gagenelaktrode 3, die Bezugselektrode 4 und die Arbeitselektroden 5_{1..} 5ₙ tauchen in den Elaktrolyt 2 ein. Es können z.B. bis zu acht Arbeitselektroden (n = 8) vorgesehen werden. Dabei bildet jede Arbeitselektrode 5ₙ mit der gemeinsamen Gegenelektrode 3 und der gemeinsamen Bezugselektrode 2 einen Dreielektrodensensor.

Die Auswerteschaltung 6 besteht aus n über die gemeinsamen Elektroden 3 und 4 miteinander gekoppelten potentiostatischen Regelkreise 7_{1..} 7ₙ. Jeder Regelkreis besteht aus zwei in Keskade geschalteten Operationsverstärkern 8ₙ und 9ₙ. Mit Hilfe des ersten Verstärkers 8ₙ wird jeweils hochohmig die Potentialdifferenz zwischen einer Arbeitselektrode 5ₙ und der gemeinsamen Bezugselektrode 4 gemessen. Diese Differenz wird vom zweiten Verstärker 9ₙ am Ausgang des ersten Verstärkers 8ₙ mit dem jeweils eingestellten Sollwert Uₙ verglichen und der Strom durch die zur Arbeitselektrode 5ₙ führende Rückkopplungsleitung 10ₙ automatisch so nachgeregelt, daß die Abweichung vom Sollwert Uₙ (Regelabweichung) minimiert wird. Die Sollwerte Uₙ und damit die Potentiale an den Arbeitselektroden 5ₙ können individuell und unabhängig voneinander eingestellt werden. Die Ströme Iₙ durch die Rückkopplungsleitungen 10ₙ fließen von den Arbeitselektroden 5ₙ durch den Elektrolyt 2 zu der gemeinsamen, geerdeten Gegenelektrode 3 ab. Da die Potentiale zwischen den Arbeitselektroden 5ₙ und der gemeinsamen Bezugselektrode 4 hochohmig gemessen werden und die gemeinsame Gegenelektrode 3 geerdet ist, arbeiten die einzelnen Potentiostatenstufen 7ₙ (potentiostatische Regelkreise) unabhängig voneinander. Die mit Hilfe der Anzeigegeräte 11_{1..} 11ₙ in den Rückkopplungsleitungen 10_{1..} 10ₙ gemessenen Ströme I_{1..} Iₙ sind ein direktes Maß für die an den Arbeitselektroden 5_{1..} 5ₙ umgesetzten Gasmengen. Anstelle der Anzeigegeräte 11_{1..} 11ₙ können auch andere Mittel zur Erfassung der in die Arbeitselektroden 5_{1..} 5ₙ fließenden Ströme z.B. elektronische Speicher, eingesetzt werden. Dabei wird das zu messende Gas gleichzeitig allen Arbeitselektroden 5_{1..} 5ₙ angeboten.

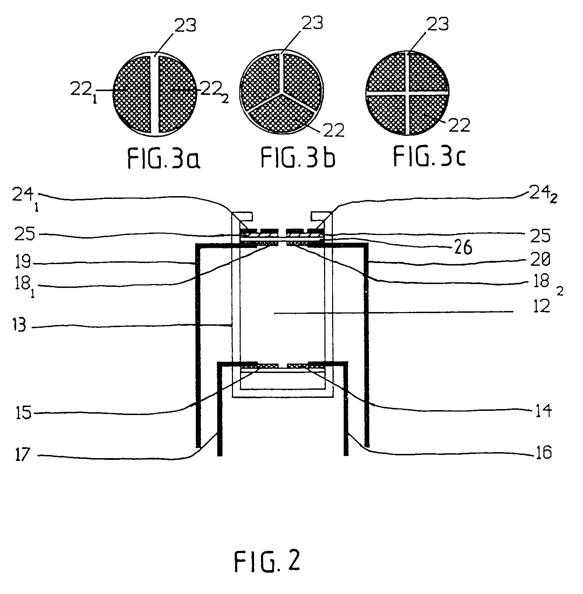

Fig. 2 zeigt die praktische Ausführung eines elektrochemischen Multielektroden-Sensors. Der Elektrolyt 12 besteht hier aus einer wäßrigen Elektrolytlösung (50%ig H₂SO₄), der von dem Gehäuse 13 umschlossen ist. Das untere Ende wird durch die Gegenelektrode 14 und die Bezugselektrode 15 (z.B. eine Pt/Luft-Elektrode) abgegrenzt. Die Gegenelektrode 14 und die Bezugselektrode 15 sind über die Anschlüsse 16, 17 herausgeführt.

Am oberen Ende sind zwei Arbeitselektroden 18₁ und 18₂ angeordnet, die mit den Zuleitungen 19 und 20 verbunden sind.

Die Arbeitselektroden sind an ihrer Außenseite, d.h. zur Gasseite hin, mit einer Diffusionsmembran 26 versehen. Über der Diffusionsmembran 26, die z.B. aus einer PTFE-Folie besteht, befindet sich ein gasdurchlässiger Abstandshalter 25, auf dem zur Einstellung und Anpassung der Empfindlichkeit Blenden 24₁ und 24₂ angeordnet sind. Die mit dem Elektrolyt 12 in Verbindung stehende Innenfläche der Arbeitselektroden 18₁, 18₂ kann katalytisch aktiviert sein. Die Auswahl geeigneter Katalysatoren, um die elektrochemische Reaktion an der Grenzfläche Arbeitselektrode/Elektrolyt gegenüber einer bestimmten Gaskomponente selektiv zu beeinflussen, ist Stand der Technik. Die Empfindlichkeit des Multielektroden-Sensors kann mit Hilfe einer vorgeschalteten Blende 21 dem jeweiligen Meßproblem angepaßt werden.

Wie in Fig. 3a dargestellt, werden durch die Arbeitselektroden 18₁ und 18₂ auf der Oberfläche des Elektrolyten 12 halbkreisförmige Meßfelder 22₁ und 22₂ gebildet, die durch einen Spalt 23 voneinander getrennt sind. Die Fig. 3b und 3c zeigen Ausführungsbeispiele für eine Sensoroberfläche mit drei bzw. vier sektorförmigen Meßfeldern für verschiedene Gaskomponenten. Zur Verbesserung der Selektivität können den Meßfeldern unterschiedliche gasspezifische Filter vorgeschaltet werden. Die Sensorkonstruktion gemäß den Fig. 2 und 3 ermöglicht einen raumsparenden und kompakten Aufbau des Multielektroden-Sensors.

### Beispiel 1

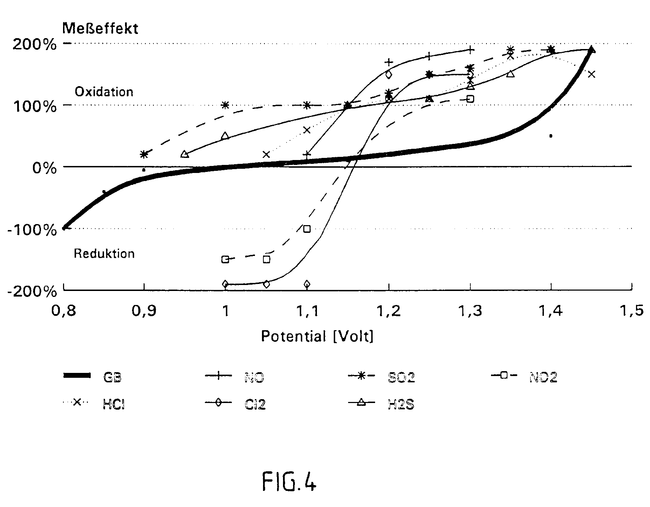

Als Beispiel wird das System Au/H₂SO₄ beschrieben, für das in Fig. 4 die potentialabhängige Empfindlichkeit für die Messung verschiedener Gase mit Hilfe einer klassischen zyklischen Voltametrie gezeigt ist. Es wird eine PTFE-Goldpulver-Gasdiffusionselektrode verwendet. Der Elektrolyt besteht aus 0,5 m Schwefelsäure. Die Messungen werden bei Zimmertemperatur und bei einer Testgasströmung von 5 l/h durchgeführt. Das Diagramm zeigt quasi stationäre Strom-Spannungskurven für die Gaskomponenten NO, SO₂, NO₂, HCl, Cl₂ und H₂S. Das sogenannte Grundbild (dick ausgezogene Kurve) wird bei reiner Luft aufgenommen. Das Potential wird gegen eine reversible H₂-Elektrode im Elektrolyt gemessen. Aus diesem mit Hilfe eines Standard-Dreielektrodensensors gemessenen Diagramms kann jeweils ein günstiges gasspezifisches Elektrodenpotential bestimmt werden. Bei der Festlegung des Potentials geht man in der Regel einen Kompromiß ein zwischen der gewünschten Empfindlichkeit und den zu unterdrückenden Querempfindlichkeiten. Der erfindungsgemäße Multielektroden-Sensor schafft die Voraussetzungen, daß einzelne Arbeiteelektroden jeweils bei den günstigsten Potentialen betrieben werden. Beispielsweise werden die Potentiale auf 1,2 V zur NO-Messung, 1,1 V zur SO₂-Messung und 1 V zur NO₂-Messung eingestellt. Da erfahrungsgemäß die Meßempfindlichkeit für alle Meßkomponenten ausreichend hoch ist, bedeutet der durch eine Verkleinerung der Meßelektrodenoberfläche entsprechend den Meßfeldern 22 (Fig. 3a bis 3c) hervorgerufene Empfindlichkeitsverlust keine Einschränkung. Die gemessenen Ströme können zum direkten Nachweis der einzelnen detektierten Gase benutzt werden. Außerdem kann man durch die parallele Messung mehrerer Komponenten die Querempfindlichkeit einer oder mehrerer Hauptkomponenten rechnerisch korrigieren.

### Beispiel 2

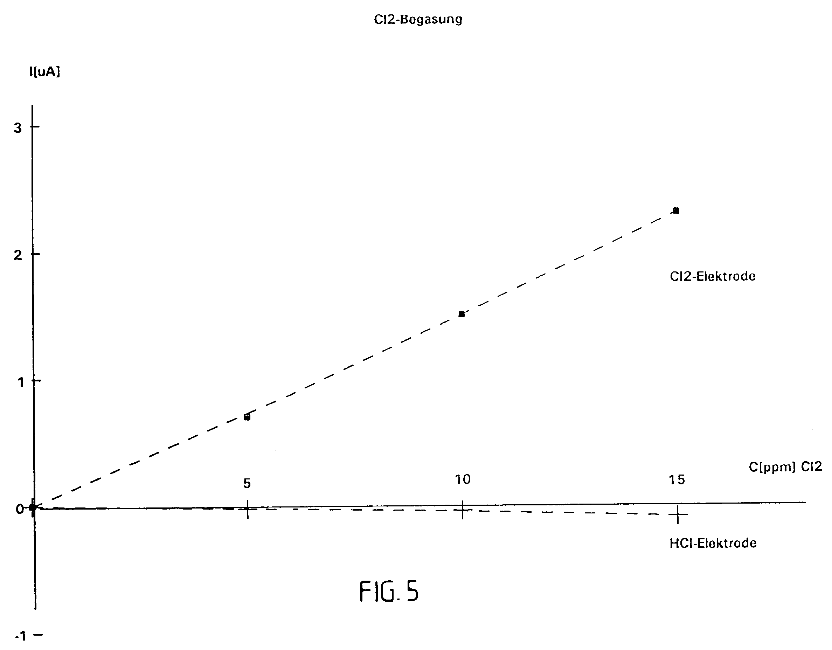

Fig. 5a zeigt die Abhängigkeit der Meßströme von einer vorgegebenen HCl-Konzentration für einen Multielektroden-Sensor mit nur zwei Arbeitselektroden. Beide Arbeitselektroden bestehen aus Gold. Die erste Arbeitselektrode wird bei einem Potential von 150 mV und die zweite Arbeitselektrode bei 0 mV gegen eine Pt/Luft-Elektrode in einem Schwefelsäurelektrolyt betrieben. Man erkennt, daß die erste Arbeitselektrode empfindlicher auf HCl reagiert. Fig. 5b zeigt die Meßströme desselben Multielektroden-Sensors gegen eine fest eingestellte Cl₂-Konzentration. Aufgrund der unterschiedlichen Arbeitselektrodenpotentiale zeigt hier die zweite Arbeitselektrode eine höhere Empfindlichkeit gegenüber Cl₂ als die erste Arbeitelektrode.

Die Selektivität kann in bekannter Weise dadurch erhöht werden, daß als Arbeitselektroden katalytisch wirksame Materialien eingesetzt werden. So kann zum Beispiel mit Hilfe einer Ruthenium-Schwarz-Arbeitselektrode, die mit einem Elektrodenpotential von 0,6 V gegen die reversible Wasserstoffelektrode betrieben wird, unter einer Platinarbeitselektrode, die auf ein Potential von 1,1 V eingestellt ist, mit Schwefelsäure als Elektrolyt ein Sensor zur simultanen und unabhängigen Messung von NO₂ und CO realisiert werden.

### Beispiel 3

Als letztes Beispiel wurde das Zeitverhalten und die Querempfindlichkeit eines Multielektrodensensors gemäß Fig. 1 für die Gase Cl₂ und HCl untersucht. Die beiden Arbeitselektroden bestehen dabei aus Goldpulver-Diffusionselektroden. Als Bezugselektrode und Gegenelektrode wurden Platin-Schwarz-Diffusionselektroden verwendet. Der Elektrolyt bestand aus 50%iger Schwefelsäure.

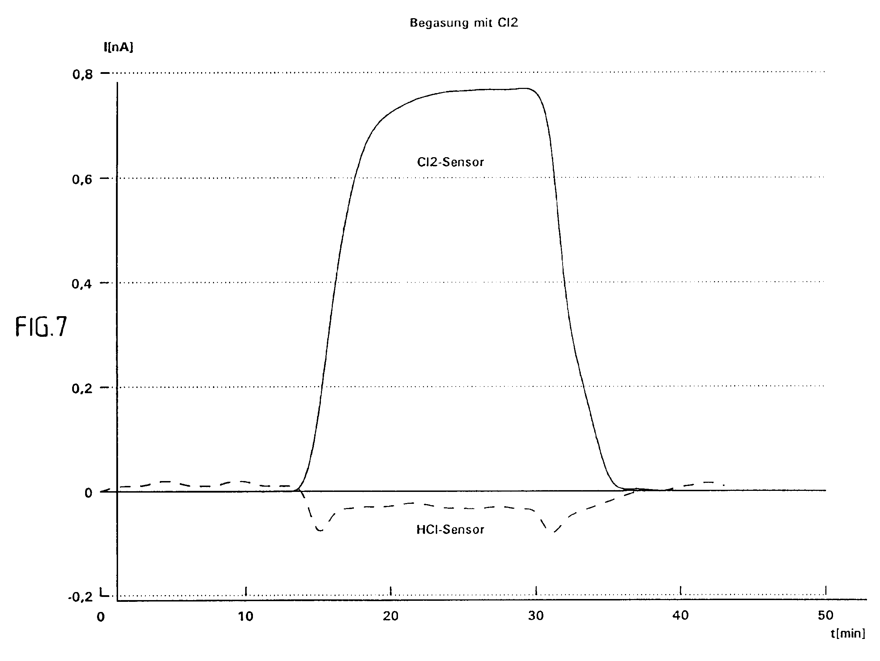

Das Potential für die chlorempfindliche Arbeitselektrode wurde auf 1000 mV und für die HCl-empfindliche Arbeitselektrode auf 1150 mV eingestellt. In Fig. 7 sind die als Funktion der Zeit simultan registrierten Verläufe der Meßsignale an der Cl₂-Arbeitselektrode und der HCl-Arbeitselektrode bei einer Begasung des Multielektrodensensors mit 5 ppm Cl₂ dargestellt, wobei das Testgas mit einem Mengenstrom von 5 l/h zugeführt wurde. Die Cl₂-Arbeitselektrode zeigt ein signifikantes Meßsignal, während die HCl-Arbeitselektrode nur eine geringe Querempfindlichkeit aufweist.

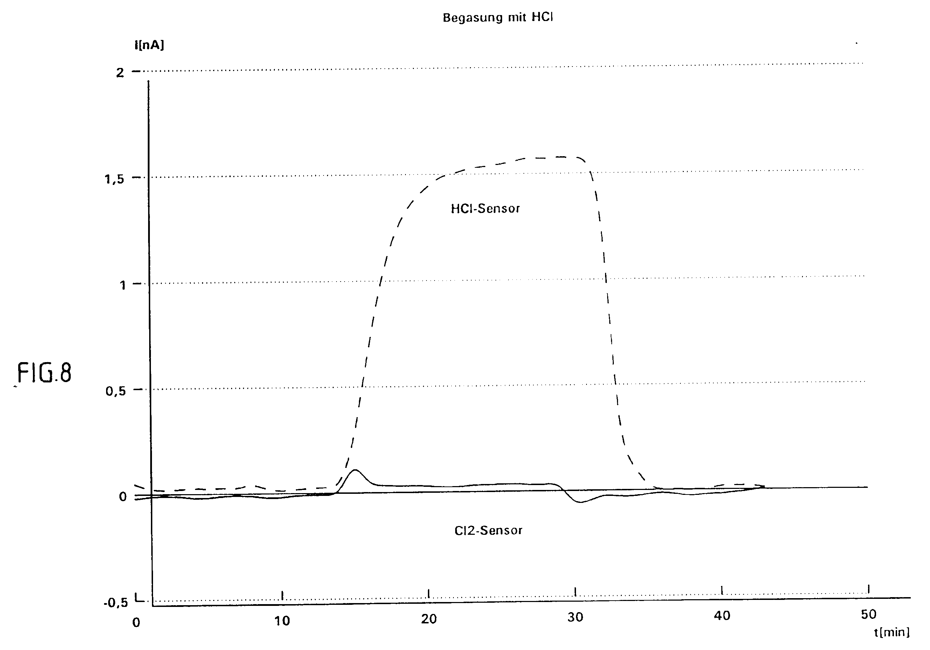

Fig. 8 zeigt in analoger Weise die Response-Kurven für die HCl-Begasung desselben Multielektrodensensors. Im übrigen wird mit den gleichen Bedingungen gearbeitet, wie bei dem Versuch nach Fig. 7. In diesem Fall zeigt die HCl-Arbeitselektrode ein signifikantes Meßsignal, während umgekehrt die Cl₂-Arbeitselektrode eine geringe Querempfindlichkeit aufweist.

## Patentansprüche

1. Vorrichtung zum simultanen Nachweis verschiedener Gaskomponenten mit einer Vielzahl von elektrochemischen Dreielektrodenmeßzellen (Arbeitselektrode-Gegenelektrode-Bezugselektrode) mit einem gemeinsamen Elektrolyt und einer potentiostatischen Auswerteschaltung zur Vorgabe und Regelung der Potentiale an den Arbeitselektroden und zur Messung der elektrochemisch erzeugten, mit den einzelnen Gaskonzentrationen korrelierten elektrischen Signale, wobei
a) die Dreielektrodenmeßzellen durch eine Vielzahl von Arbeitselektroden mit einer gemeinsamen Gegenelektrode (3) und einer gemeinsamen Bezugselektrode (4) gebildet werden,
b) die potentiostatische Auswerteschaltung (6) Regelkreise (7_{1..} 7ₙ) enthält, die die Potentiale der Arbeitselektroden (5_{1..} 5ₙ), bezogen auf die Bezugselektrode (4) einzeln und unabhängig voneinander konstant halten und
c) die Auswerteschaltung (6) Mittel (11_{1..} 11ₙ) zur Erfassung der in die Arbeitselektroden (5_{1..} 5ₙ) fließenden, den Gaskonzentrationen entsprechenden Stromsignale (I_{1..} Iₙ) aufweist.

2. Vorrichtung nach Anspruch 1, dadurch gekennzeichnet, daß ein Regelkreis (7ₙ) jeweils aus zwei in Kaskade geschalteten Operationsverstärkern (8ₙ und 9ₙ) besteht, wobei der erste Verstärker (8ₙ) die Potentialdifferenz zwischen einer Arbeitselektrode Aₙ und der Bezugselektrode (4) hochohmig abgreift und der zweite Verstärker (9ₙ) diese Potentialdifferenz am Ausgang des ersten Verstärkers (8ₙ) mit einem voreingestellten Sollwert Uₙ vergleicht und den vom Ausgang des zweiten Verstärkers (9ₙ) zur Arbeitselektrode (5ₙ) fließenden Strom Iₙ so nachregelt, daß die Abweichung vom Sollwert Uₙ minimiert wird.

3. Vorrichtung nach Ansprüchen 1 und 2, dadurch gekennzeichnet, daß der Elektrolyt (12) aus einer Elektrolyt-Lösung besteht und die Arbeitselektrode (18₁, 18₂) in Form von räumlich getrennten Meßfeldern (22₁, 22₂) auf einer gemeinsamen Membran den Elektrolyt zur Gasseite hin begrenzen, während die Gegenelektrode (14) und die Bezugselektrode (15) auf der gegenüberliegenden Seite des Elektrolyten (12) angeordnet ist.

4. Vorrichtung nach Ansprüchen 1 bis 3, dadurch gekennzeichnet, daß den Arbeitselektroden (5ₙ, 18₁, 18₂) bzw. den Meßfeldern (22) gasspezifische Filter vorgeschaltet sind.

5. Vorrichtung nach Ansprüchen 1 bis 4, dadurch gekennzeichnet, daß die Arbeitselektroden (5ₙ, 18₁, 18₂) entsprechend den zu messenden Gaskomponenten in unterschiedlicher Weise katalytisch aktiviert sind.

6. Vorrichtung nach Ansprüchen 1 bis 5, dadurch gekennzeichnet, daß die Meßempfindlichkeit der Arbeitselektroden (5ₙ, 18₁, 18₂) durch Vorschaltung von Blenden (24₁, 24₂) oder Diffusionsmembranen (26) unterschiedlich einstellbar ist.

7. Vorrichtung nach Ansprüchen 1 bis 6, dadurch gekennzeichnet, daß zur Messung von HCl und Cl der Elektrolyt aus wäßriger Schwefelsäure und die Arbeitselektroden aus Gold bestehen.

## Claims

1. Device for the simultaneous detection of dissimilar gas components with a number of electrochemical, three-electrode measuring cells (working electrode/counter-electrode/reference electrode) with a common electrolyte and a potentiostatic evaluation circuit for setting and adjusting the potentials at the working electrodes and for measuring the electrochemically-produced electrical signals correlated with the individual gas concentrations, wherein
a) the three-electrode measuring cells are formed by a number of working electrodes with a common counter-electrode (3) and a common reference electrode (4);
b) the potentiostatic evaluation circuit (6) contains control loops (7₁ .. 7ₙ) that maintain the potentials of the working electrodes (5₁ .. 5ₙ) constant with respect to the reference electrode (4), both individually and independently of each other ; and
c) the evaluation circuit (6) contains means (11₁ ..11ₙ) for detecting the electric current signals (I₁ .. Iₙ) corresponding to the gas concentrations, which are flowing in the working electrodes (5₁ .. 5ₙ).

2. Device according to claim 1, characterised in that each control loop (7ₙ) consists of two operational amplifiers (8ₙ and 9ₙ) connected in cascade, whereby a high-resistance measurement of the potential-difference between a working electrode Aₙ and the reference electrode (4) is made by means of the first amplifier (8ₙ), and the second amplifier (9ₙ) compares this difference at the output of the first amplifier (8ₙ) with a preset set-point Uₙ and the current Iₙ flowing from the output of the second amplifier (9ₙ) to the working electrode (5ₙ) is adjusted so that the deviation from the set-point Uₙ is minimized.

3. Device according to claims 1 and 2, characterised in that the electrolyte (12) consists of an electrolyte solution, and the working electrodes (18₁, 18₂) delimit the electrolyte on the gas side in the form of physically-separate measuring fields (22₁, 22₂) on a common membrane, whereas the counter-electrode (14) and the reference electrode (15) is arranged on the opposite side of the electrolyte (12).

4. Device according to claims 1 to 3, characterised in that the gas-specific filters are connected in series with the working electrodes (5ₙ, 18₁, 18₂) and the measuring fields (22) respectively.

5. Device according to claims 1 to 4, characterised in that the working electrodes (5ₙ, 18₁, 18₂) are activated catalytically in different ways according to the gas components being measured.

6. Device according to claims 1 to 5, characterised in that the measuring sensitivity of the working electrodes (5ₙ, 18₁, 18₂) can be adjusted independently by connecting orifices (24₁, 24₂) or diffusion membranes (26) in series.

7. Device according to claims 1 to 6, characterised in that for the measurement of HCl and Cl the electrolyte consists of aqueous sulphuric acid and the working electrodes consist of gold.

## Revendications

1. Appareil pour la détermination simultanée de composants différents d'un gaz à l'aide de plusieurs cellules de mesure électrochimiques à trois électrodes (électrode de travail, contre-électrode, électrode de référence) comportant un électrolyte commun et un circuit d'évaluation de potentiels pour fournir et commander les potentiels sur les électrodes de travail et pour mesurer les signaux électriques obtenus de façon électrochimique et corrélés aux concentrations des composants du gaz, dans lequel :
a) les cellules de mesure à trois électrodes sont formées de plusieurs électrodes de travail avec une contre-électrode commune (3) et une électrode de référence commune (4) ;
b) le circuit d'évaluation de potentiels (6) comporte des circuits de réglage (7₁...7ₙ) qui maintiennent constants et indépendants les uns des autres les potentiels individuels des électrodes de travail (5₁...5ₙ) par rapport à l'électrode de référence (4), et
c) le circuit d'évaluation (6) présente des moyens (11₁...11ₙ) pour déterminer les signaux de courant (I₁...Iₙ) parcourant les électrodes de travail (5₁...5ₙ) et correspondant aux concentrations de gaz.

2. Appareil selon la revendication 1, caractérisé en ce que chaque circuit de réglage (7ₙ) se compose de deux amplificateurs opérationnels (8ₙ et 9ₙ) connectés en cascade, dans lequel le premier amplificateur (8ₙ) reçoit à haute impédance la différence de potentiel entre une électrode de travail Aₙ et l'électrode de référence (4), et dans lequel le deuxième amplificateur (9ₙ) compare cette différence de potentiel en sortie du premier amplificateur (8ₙ) à une valeur de consigne prédéterminée Uₙ et règle le courant In allant de la sortie du deuxième amplificateur (9ₙ) à l'électrode de travail (5ₙ) de sorte que l'écart à la valeur prédéterminée Uₙ est minimal.

3. Appareil selon les revendications 1 et 2, caractérisé en ce que l'électrolyte (12) est formé par une solution électrolytique et en ce que les électrodes de travail (18₁, 18₂) en forme de champs de mesure (22₁, 22₂) spatialement séparés limitent l'électrolyte côté gaz sur une membrane commune, tandis que la contre-électrode (14) et l'électrode de référence (15) sont agencées sur le côté opposé de l'électrolyte (12).

4. Appareil selon les revendications 1 à 3, caractérisé en ce que des filtres spécifiques au gaz sont connectés aux électrodes de travail (5ₙ, 18₁, 18₂), respectivement aux champs de mesure (22).

5. Appareil selon les revendications 1 à 4, caractérisé en ce que les électrodes de travail (5ₙ, 18₁, 18₂) sont activées catalytiquement de manière différente selon les composants du gaz à mesurer.

6. Appareil selon les revendications 1 à 5, caractérisé en ce que la sensibilité de mesure des électrodes de travail (5ₙ, 18₁, 18₂) est réglable de manière différente par installation d'écrans (24₁, 24₂) ou de membranes de diffusion (26).

7. Appareil selon les revendications 1 à 6, caractérisé en ce que, pour la mesure de HCl et Cl, l'électrolyte est une solution d'acide sulfurique et les électrodes de travail sont en or.
